# EUROPEAN PATENT APPLICATION

(11) **EP 1 982 703 A2**
(43) Date of publication of application: **22.10.2008**
(21) Application number: 08007650.8
(22) Date of filing: 18.04.2008
(51) Int. Cl.: A61K 9/72, A61K 9/51

(54) **A transpulmonary composition**

(30) Priority: 19.04.2007 JP 2007110038
(71) Applicant: FUJIFILM Corporation, Minato-ku Tokyo (JP)
(72) Inventor: Ooya, Shouji, Ashigarakami-gu Kanagawa 258-8538 (JP); Aimi, Makiko, Ashigarakami-gu Kanagawa 258-8538 (JP); Ogiwara, Kazutaka, Ashigarakami-gu Kanagawa 258-8538 (JP)
(74) Representative: HOFFMANN EITLE

(57) **Abstract**

It is an object of the present invention to provide a transpulmonary preparation wherein physiologically active ingredient can be stably retained and which has high absorption efficacy and gives low damage to tissue. The present invention provides a transpulmonary preparation which comprises protein nanoparticles containing an active ingredient and having an average particle size of 200 nm to 500 nm.

## Description

### Technical Field

The present invention relates to a transpulmonary preparation which comprises protein nanoparticles.

### Background Art

When a protein preparation such as insulin is orally administered, there is a problem that it is decomposed in stomach, and its intestinal absorption is low. Therefore, a protein preparation is usually administered via intravenous injection. However, it is necessary for a diabetes patient who requires daily intravenous injection to develop a administration route having high compliance.

Recently, administration route of drugs has been extensively studied, and preparations which utilize skin or mucosa as administration route such as transdermal and transpulmonary administration have been studied. These administration methods have advantages that bioavailability is high; compliance of patients is high; stop of administration at excessive administration is easy; and administration to a physically-challenged patient is easy. A transpulmonary preparation of insulin powder (Exubera) is marketed in view of these advantages, and effectiveness of these administration routes is shown.

The absorption rate of an active ingredient of a preparation in transpulmonary administration depends on inhalation efficacy, stability of an active ingredient, and absorption efficacy of a preparation in lung. In conventional transpulmonary preparation, an active ingredient per se is powdered into a size of several µ m which shows high natural inhalation efficacy. Therefore, decomposition or degeneration of an active ingredient may occur, and there is a problem in the absorption efficacy in lung.

Recently, the effectiveness of lung as administration route has been shown. Therefore, development of a preparation as well as development of inhalation of preparation has been extensively studied, and focus is drawn to increase of absorption efficacy in lung. It is important to develop a preparation by which unstable active ingredient can be stably administered with high absorption efficacy and without damage to tissue.

In the drug delivery system (DDS) field, use of nanoparticles has been expected, and nanoparticles have been used as carriers of drug or gene. In particular, research using polymeric micelles has been actively carried out (Japanese Patent Publication (kokai) 10-218763. In many cases, AB or ABA type block copolymers are employed because of their simple structures. Polymeric micelles are characterized by large drug loading capacity, high water solubility, high structural stability, nonaccumulativeness, small particle diameters (not greater than 100 nm), and functional separability. Thus, research aiming at the targeting of a target site or at the solubilization of hydrophobic drugs has been conducted. However, synthetic polymer generally has a problem of biocompatibility, and thus administration into lung which has weak biological protection function involves, a danger. Japanese Patent Publication (kokai) 2001-172203 discloses use of cationized gelatin particles as a transpulmonary absorption agent of polymer drug, and its average particle size is about 3.0 µm.

### Disclosure of the Invention

It is an object of the present invention to provide a transpulmonary preparation wherein physiologically active ingredient can be stably retained and which has high absorption efficacy and gives low damage to tissue.

As a result of intensive studies in order to achieve the above object, the present inventors found that a transpulmonary preparation wherein physiologically active ingredient can be stably retained and which has high absorption efficacy and gives low damage to tissue can be provided by encapsulating a physiologically active substance into protein nanoparticle.

Thus, the present invention provides a transpulmonary preparation which comprises protein nanoparticles containing an active ingredient and having an average particle size of 200 nm to 500 nm.

Preferably, the active ingredient is a drug.

Preferably, the drug is a therapeutic agent for diabetes, a polypeptide, an antioxidant, an antibody, or a nucleic acid.

Preferably, the drug is insulin or interferon.

Preferably, the protein is casein, collagen, gelatin, or albumin.

Preferably, the protein is casein.

According to the present invention, (1) a physiologically active substance having low stability can be stably administered, (2) absorption efficacy in lung is high, and (3) a physiologically active substance can be absorbed via lung without damage by using natural material such as casein or gelatin.

### Brief Description of the Drawings

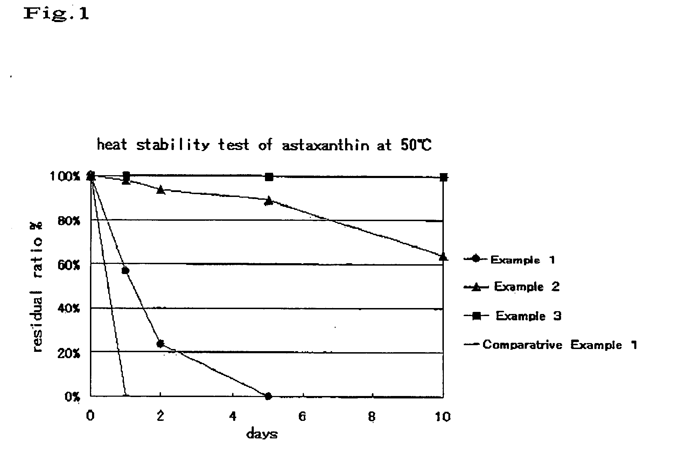

Fig. 1 shows the result of heat stability test of astaxanthin in the nanoparticles of Examples 1 to 3 and the emulsion of Comparative example.

### Best Mode for Carrying Out the Invention

Recently, in the development of preparation, development of a new drug as well as development of a new administration route is studied. In particular, transpulmonary administration is expected as a useful administration route, since first pass effect can be avoided and bioavailability is high.

The absorption rate of an active ingredient of a preparation in transpulmonary administration depends on inhalation efficacy, stability of an active ingredient, and absorption efficacy of a preparation in lung. In view of the inhalation efficacy and the absorption efficacy in lung, protein nanoparticles having an average particle size of 200 nm to 500 nm is used in the transpulmonary preparation of the present invention. The stability of an active ingredient can be increased by encapsulating the active ingredient into protein. The ratio of the active ingredient to the protein is not particularly limited, so long as the present invention can be carried out. This ratio is preferably 0.0001% by weight to 100% by weight, more preferably 0.001% by weight to 50% by weight, further more preferably 0.01% by weight to 30% by weight.

The active ingredients which are encapsulated into protein may be any of a drug, a cosmetic component, and a supplement component, and is preferably a drug. The examples of the drug may include antibiotics, antioxidants, anti-cancer agents, immune-suppressing agents, blood-making agents, antithrombotic agents, therapeutic agents for infection disease, muscle relaxants, antidepressants, combination cold remedies, anti-viral agents, anti-tumor agents, antipyretic agents, analgesic agents, anesthetic agents, antiflash agents, antiulcerogenic agents, antiallergenic agents, hormone agents, antiepilepsy agents, antipsychotic agents, anti-dementia agents, antiparkinson agents, hypnotic sedative agents, cardiotonic agents, antiarrhythmic agents, vasodilating agents, vasoconstrictive agents, antihypertensive agents such as antihypertensive diuretic agents, therapeutic agents for diabetes, anticoagulants, hypocholesterolemic agents, therapeutic agents for osteoporosis, hyperlipidemia agent, respiratory stimulants, cough medicines, vitamins, vaccines, growth factors, antisense oligonucleotides, polypeptides, antibodies, and nucleic acids, but are not limited thereto. Preferably, the drug may be therapeutic agents for diabetes, polypeptides, antioxidants, antibodies, nucleic acids, growth factors, or anti-cancer agents. More preferably, the drug may be therapeutic agents for diabetes, polypeptides, or hormones. Further preferably, the drug may be insulin, interferon, erythropoietin, or follicle-stimulating hormone.

The type of protein in the protein nanoparticles is not particularly limited. However, a protein having a molecular weight of approximately 1,000 to 1,000,000 is preferably used. Specific examples of a protein that can be used include, but are not limited to, casein, collagen, gelatin, albumin, fibrin, fibroin, transferrin, laminin, fibronectin, vitronectin and globulin. Among them, preferred examples are casein, collagen, gelatin, and albumin. More preferred examples are casein, gelatin, and collagen. Most preferred example is casein. The origin of the protein is not particularly limited. Thus, any human, bovine, swine, bird, fish or plant protein, as well as recombinant protein, can be used. Examples of recombinant gelatin that can be used include, but are not limited to, gelatins described in EP0926543A, EP1014176 A, US Patent No. 6,992,172, EP1398324A and WO2004/085473.

Further, the protein may be partially hydrolyzed. The gelatin may have amino acid identity of 40%, preferably 50% or more, more preferably 80% or more most preferably 90% or more, with the sequence of collagen derived from living organisms. The collagens herein may be any naturally-occurring one, and are preferably type I, type II, type III, type IV or type V collagen.

The origin of the casein used in the present invention is not particularly limited. Casein may be milk-derived or bean-derived. Any of α-casein, β-casein, γ-casein, and κ-casein, as well as a mixture of any thereof, can be used. Also, a recombinant thereof can be used. Preferably, casein sodium can be used. Caseins may be used alone or in combinations of two or more. The casein may be a salt.

The protein may be subjected to crosslinking treatment. The crosslinking treatment may be carried out by heat, light, a crosslinking agent or an enzyme. The crosslinking by polyion complex and hydrophobic interaction may be used. Preferably, the crosslinking treatment may be carried out by a crosslinking agent or an enzyme. More preferably, the crosslinking treatment may be carried out by am enzyme.

In the crosslinking by heat, a protein is treated by heat so that the protein is crosslinked. The crosslinking is carried out at 50 to 200°C. If the temperature is lower than 50°C, crosslinking become insufficient or cannot be carried out. If the temperature is higher than 200°C, denaturation of protein becomes significant. In view of factors of production and activity, the temperature is preferably 60 to 180°C, and most preferably 90 to 150°C.

In the crosslinking by light, a protein is irradiated with radiation, for example, so that the protein is crosslinked. Specifically, the protein is irradiated with physical energy such as ultraviolet, electron beam, or gamma beam, so that physical crosslinking is generated.

Inorganic or organic crosslinking agents can be used as a crosslinking agent. Specific examples of inorganic or organic crosslinking agents include, but not limited to, chromium salts (chrome alum, chromium acetate, etc.); calcium salts (calcium chloride, calcium hydroxide, etc.); aluminum salt (aluminum chloride, aluminum hydroxide, etc.); carbodiimides (EDC, WSC, N-hydroxy-5-norbornene-2,3-dicarboxyimide (HONB), N-hydroxysuccinic acid imide (HOSu), dicyclohexylcarbodiimide (DCC), etc.); N-hydroxysuccinimide; phosphorus oxychloride; and vinylsulfone. The aforementioned crosslinking agent may be used alone or in combination of two or more types. When a crosslinking agent is used, it can be added in a weight that is 0.1 % to 100% by weight of the protein weight, so that crosslinking treatment can be carried out.

Further, a protein having a reactive group introduced therein may be used. The reactive group may be a light-reactive group (for example, cinnamyl group), a radical-generating group (for example, dithiocarbamyl group, camphorquinone group), and a vinyl group (for example, styrene group). The protein may be used in combination with a compound which can react with the reactive group.

When crosslinking by enzyme is carried out, the enzyme is not particularly limited as long as it has action for crosslinking protein. Preferably, transglutaminase or laccase is used. Most preferably, transglutaminase is used for crosslinking. Specific examples of protein which is enzymatically crosslinked by transglutaminase are not particularly limited as long as the protein contains a lysine residue or a glutamine residue. Transglutaminase may be derived from a mammal or a microorganism. Specific examples thereof include the Activa series by Ajinomoto Co., Inc., commercially available mammalian-derived transglutaminase serving as a reagent, such as guinea pig liver-derived transglutaminase, goat-derived transglutaminase, or rabbit-derived transglutaminase, produced by, for example, Oriental Yeast Co., Ltd., Upstate USA Inc., and Biodesign International.

The amount of an enzyme used for the crosslinking treatment can be adequately determined depending upon protein type. In general, an enzyme can be added in a weight that is 0.1% to 100% and preferably approximately 1% to 50% of the protein weight.

The method for producing the nanoparticles used in the present invention is not particularly limited. For example, casein nanoparticles containing an active ingredient therein, which are prepared by the following steps (a) to (c), can be used:
(a) mixing casein with a basic aqueous medium at pH of 8 to less than 11;
(b) adding at least one active ingredient to the solution obtained in step (a); and
(c) injecting the solution obtained in step (b) into an acidic aqueous medium at a pH of 3.5 to 7.5.

Further, as another embodiment, casein nanoparticles containing an active ingredient therein, which are prepared by the following steps (a) to (c), can be used:
(a) mixing casein with a basic aqueous medium at a pH of 8 to less than 11;
(b) adding at least one active ingredient to the solution obtained in step (a); and
(c) lowering the pH of the solution obtained in step (b) to a pH value which is distinct from the isoelectric point by 1 unit or more.

The agent of the present invention may further comprise an additive depending on its use. Example of additive may include pH adjusters (for example, sodium citrate, sodium acetate, sodium hydroxide, potassium hydroxide, and phosphoric acid), various salts, polysaccharides (for example, hyaluronic acid, heparin, heparin sulfate, and chondroitin sulfate), and sugar (for example, glucose), but are not limited thereto.

The dose of the transpulmonary preparation which comprises protein nanoparticles of the present invention can be adequately determined depending upon type and amount of active ingredient and upon user weight and condition, for example. The dose for single administration is generally approximately 10 µg to 100 mg/kg and preferably 10 µg to 50mg/kg.

The present invention is hereafter described in greater detail with reference to the following examples, although the technical scope of the present invention is not limited thereto.

### Examples

### Example 1:

Milk-derived casein (15 mg; Wako Pure Chemical Industries, Ltd.) was mixed with phosphate buffer (pH 9, 1.5 mL). Astaxanthin (9 mg: Wako Pure Chemical Industries, Ltd.) was dissolved in ethanol (1 mL). The two different solutions were mixed together. After ethanol was evaporated, the resulting liquid mixture (1mL) was injected into phosphate buffer water (pH 5, 10 mL) with the use of microsyringe at an external temperature of 40°C during stirring at 800 rpm. Thus, casein nanoparticles were obtained. The average particle size of the above particles was measured with a "Microtrac" light scattering photometer (NIKKISO Co., Ltd.) and found to be 274 nm.

### Example 2:

Milk-derived casein (15 mg; Wako Pure Chemical Industries, Ltd.) was mixed with phosphate buffer (pH 9, 1.5 mL). Astaxanthin (9 mg: Wako Pure Chemical Industries, Ltd.) and tocopherol (2.75mg) were dissolved in ethanol (1 mL). The two different solutions were mixed together. After ethanol was evaporated, the resulting liquid mixture (1mL) was injected into phosphate buffer water (pH 5, 10 mL) with the use of microsyringe at an external temperature of 40°C during stirring at 800 rpm. Thus, casein nanoparticles were obtained. The average particle size of the above particles was measured with a "Microtrac" light scattering photometer (NIKKISO Co., Ltd.) and found to be 293 nm.

### Example 3:

Milk-derived casein (15 mg; Wako Pure Chemical Industries, Ltd.) was mixed with phosphate buffer (pH 9, 1.5 mL). Astaxanthin (9 mg: Wako Pure Chemical Industries, Ltd.) and tocopherol (2.75mg) were dissolved in ethanol (1 mL). The two different solutions were mixed together. After ethanol was evaporated, the resulting liquid mixture (1mL) was injected into phosphate buffer water (pH 5, 10 mL) with the use of microsyringe at an external temperature of 40°C during stirring at 800 rpm. Thus, casein nanoparticles were obtained. The average particle size of the above particles was measured with a "Microtrac" light scattering photometer (NIKKISO Co., Ltd.) and found to be 293 nm. Ascorbic acid (100mg) was added to this dispersion liquid.

### Test Example 1:

The casein nanoparticles prepared in Examples 1, 2 and 3 were left in thermostat bath at 50°C, and time course stability test was carried out for 10 days. In Comparative example, astaxanthin olive oil emulsion was used. The amount of astaxanthin was calculated from absorption spectra (Abs.500nm) (Fig.1). By incorporating astaxanthin having low stability into casein nanoparticles, the stability of astaxanthin can be increased. Further, its effect can be increased by addition of an additive.

### Example 4:

Acid-treated gelatin (20 mg), chondroitin sulfate-C (1 mg), transglutaminase preparation (10 mg; Activa TG-S, Ajinomoto Co., Inc.), adriamycin (0.4mg; doxorubicin hydrochloride, Wako Pure Chemical Industries, Ltd.) and deionized water (1ml) were mixed together. The resultant solution (1 ml) was injected into ethanol (10 mL) with the use of a microsyringe at an external temperature of 40°C during stirring at 800 rpm. The resultant dispersion liquid was allowed to stand at an external temperature of 55°C for 5 hours, so that crosslinked gelatin nanoparticles were obtained, The average particle size of the above particles was measured with a light scattering photometer (DLS-7000; Otsuka Electronics Co., Ltd.) and found to be 115 nm. The nanoparticle dispersion liquid was centrifuged, and the supernatant ethanol was discarded. A physiological saline (10ml) was added thereto, and the particles were dispersed again. The incorporation ratio of adriamycin was calculated from the absorption spectra (Abs.480nm) of the supernatant ethanol, and was found to be 98%. The average particle size after redispersion was measured with a light scattering photometer (DLS-7000; Otsuka Electronics Co., Ltd.) and found to be 345 nm.

## Claims

1. A transpulmonary preparation which comprises protein nanoparticles containing an active ingredient and having an average particle size of 200 nm to 500 nm.

2. The transpulmonary preparation according to claim 1, wherein the active ingredient is a drug.

3. The transpulmonary preparation according to claim 2, wherein the drug is a therapeutic agent for diabetes, a polypeptide, an antioxidant, an antibody, or a nucleic acid.

4. The transpulmonary preparation according to claim 2, wherein the drug is insulin or interferon.

5. The transpulmonary preparation according to claim 1, wherein the protein is casein, collagen, gelatin, or albumin.

6. The transpulmonary preparation according to claim 1, wherein the protein is casein.
